# EUROPÄISCHE PATENTSCHRIFT

(11) **EP 3 036 081 B1**
(45) Veröffentlichungstag und Bekanntmachung des Hinweises auf die Patenterteilung: **20.05.2020**
(21) Anmeldenummer: 14759098.8
(22) Anmeldetag: 13.08.2014
(51) Int. Cl.: B29C 49/46, B29C 49/12, B29C 49/42, B29C 49/58, B29C 49/36

(54) **VERFAHREN UND VORRICHTUNG ZUR BLASFORMENDEN HERSTELLUNG VON ZUMINDEST BEREICHSWEISE STERILEN BEHÄLTERN**
METHOD AND DEVICE FOR BLOW-MOLDING CONTAINERS WHICH ARE STERILE AT LEAST IN SOME AREAS
PROCÉDÉ ET DISPOSITIF PERMETTANT DE PRODUIRE PAR MOULAGE PAR SOUFFLAGE DES CONTENANTS STÉRILES AU MOINS PAR ENDROITS

(30) Priorität: 19.08.2013 DE 102013013591
(43) Veröffentlichungstag der Anmeldung: 29.06.2016
(73) Patentinhaber: KHS Corpoplast GmbH, 22145 Hamburg (DE)
(72) Erfinder: LEWIN, Frank, 22889 Tangstedt (DE); HEROLD, Thomas, 22941 Bargteheide (DE); MEYER, Jan, Fabian, 22337 Hamburg (DE); GERHARDS, Martin, 22089 Hamburg (DE); KLATT, Dieter, 22147 Hamburg (DE); BAUMGARTE, Rolf, 22926 Ahrensburg (DE)
(74) Vertreter: Hausfeld, Norbert
(86) Internationale Anmeldenummer: PCT/EP2014/002221
(87) Internationale Veröffentlichungsnummer: WO 2015/024642

(56) Entgegenhaltungen:
- EP-A1- 1 258 336
- EP-A2- 2 283 991
- EP-A2- 2 295 223
- EP-B1- 2 483 052
- DE-A1-102007 017 938

## Beschreibung

Die Erfindung betrifft ein Verfahren zur Herstellung von blasgeformten, mindestens bereichsweise sterilen Behältern, bei dem ein Vorformling aus einem thermoplastischen Material zunächst erwärmt und dann in einer Blasstation von einer Reckstange gereckt und mittels einer Blasdüse mit einem unter Druck stehenden Fluid beaufschlagt wird, sowie bei dem eine Sterilisationseinrichtung in der Blasstation angeordnet wird.

Darüber hinaus betrifft die Erfindung eine Vorrichtung zur Herstellung von blasgeformten, mindestens bereichsweise sterilen Behältern, die mit einer Heizstrecke zur Temperierung der Vorformlinge und mit einer Blasstation zur Blasverformung der Vorformlinge in die Behälter versehen ist, wobei die Blasstation eine Reckstange aufweist, sowie bei der eine Sterilisationseinrichtung in der Blasstation angeordnet ist
Eine Herstellung von sterilen, blasgeformten Behältern erfolgt typischerweise derart, dass diese Behälter nach ihrer Blasformung und vor einer Befüllung unter Verwendung von Wasserstoffperoxid oder anderen Chemikalien sterilisiert werden. Ebenfalls ist es bereits bekannt, die bei der Blasformung der Behälter als Ausgangsprodukt verwendeten Vorformlinge zu sterilisieren, insbesondere den Bereich der inneren Oberfläche dieser Vorformlinge.

Bei einer Behälterformung durch Blasdruckeinwirkung werden Vorformlinge aus einem thermoplastischen Material, beispielsweise Vorformlinge aus PET (Polyethylenterephtalat), innerhalb einer Blasmaschine unterschiedlichen Bearbeitungsstationen zugeführt. Typischerweise weist eine derartige Blasmaschine eine Heizeinrichtung sowie eine Blasstation auf, in deren Bereich der zuvor temperierte Vorformling durch biaxiale Orientierung zu einem Behälter expandiert wird. Die Expansion erfolgt mit Hilfe von Druckluft, die in den zu expandierenden Vorformling mittels einer Blasdüse eingeleitet wird. Der verfahrenstechnische Ablauf bei einer derartigen Expansion des Vorformlings wird in der DE-OS 43 40 291 erläutert.

Der grundsätzliche Aufbau einer Blasstation zur Behälterformung wird in der DE-OS 42 12 583 beschrieben. Möglichkeiten zur Temperierung der Vorformlinge werden in der DE -OS 23 52 926 erläutert.

Innerhalb der Vorrichtung zur Blasformung können die Vorformlinge sowie die geblasenen Behälter mit Hilfe unterschiedlicher Handhabungseinrichtungen transportiert werden. Bewährt hat sich insbesondere die Verwendung von Transportdornen, auf die die Vorformlinge aufgesteckt werden. Die Vorformlinge können aber auch mit anderen Trageinrichtungen gehandhabt werden. Die Verwendung von Greifzangen zur Handhabung von Vorfomlingen und die Verwendung von Spreizdornen, die zur Halterung in einen Mündungsbereich des Vorformlings einführbar sind, gehören ebenfalls zu den verfügbaren Konstruktionen.

Eine Handhabung von Behältern unter Verwendung von Übergaberädern wird beispielsweise in der DE-OS 199 06 438 bei einer Anordnung des Übergaberades zwischen einem Blasrad und einer Ausgabestrecke beschrieben.

Die bereits erläuterte Handhabung der Vorformlinge erfolgt zum einen bei den sogenannten Zweistufenverfahren, bei denen die Vorformlinge zunächst in einem Spritzgussverfahren hergestellt, anschließend zwischengelagert und erst später hinsichtlich ihrer Temperatur konditioniert und zu einem Behälter aufgeblasen werden. Zum anderen erfolgt eine Anwendung bei den sogenannten Einstufenverfahren, bei denen die Vorformlinge unmittelbar nach ihrer spritzgusstechnischen Herstellung und einer ausreichenden Verfestigung geeignet temperiert und anschließend aufgeblasen werden.

Im Hinblick auf die verwendeten Blasstationen sind unterschiedliche Ausführungsformen bekannt. Bei Blasstationen, die auf rotierenden Transporträdern angeordnet sind, ist eine buchartige Aufklappbarkeit der Formträger häufig anzutreffen. Es ist aber auch möglich, relativ zueinander verschiebliche oder andersartig geführte Formträger einzusetzen. Bei ortsfesten Blasstationen, die insbesondere dafür geeignet sind, mehrere Kavitäten zur Behälterformung aufzunehmen, werden typischerweise parallel zueinander angeordnete Platten als Formträger verwendet.

Hinsichtlich der Sterilisierung von Vorformlingen sind aus dem Stand der Technik bereits unterschiedliche Verfahren und Vorrichtungen bekannt, die jedoch alle verfahrensspezifische Nachteile aufweisen, die einer zuverlässigen Sterilisierung der Vorformlinge bei gleichzeitig hohen Durchsatzraten entgegenstehen.

In der EP-A 1 086 019 wird beispielsweise die Sterilisierung von heißen Vorformlingen mit einem heißen gasförmigen Sterilisationsmittel beschrieben. Es werden hintereinander angeordnete separate Behandlungsstationen verwendet, nämlich ein erstes Heizmodul, ein Sterilisiermodul sowie ein zweites Heizmodul. Nachteilig ist hierbei das Temperaturverhalten des Vorformlings während des Sterilisiervorganges sowie das unkontrollierte Austreten des Sterilisationsmittels aus dem Vorformling innerhalb der Heizung.

In der EP-A 1 896 245 wird ein Verfahren beschrieben, bei dem vor der Heizung ein gasförmiges Sterilisationsmittel in einen kalten Vorformling eingeleitet wird und hier kondensiert. Problematisch ist hier die Sicherstellung einer vollständigen Kondensatbildung auf der gesamten Innenfläche des Vorformlings, da das einströmende heiße Sterilisationsmittel die Innenwandtemperatur des Vorformlings erhöht. Darüber hinaus tritt auch hier das Sterilisationsmittel nach seiner Verdampfung im Bereich der Heizung unkontrolliert innerhalb der Heizung aus dem Vorformling aus.

In der EP-A 2 138 298 wird eine Vorrichtung beschrieben, bei der vorsorglich sowohl vor dem verwendeten Blasmodul als auch hinter dem verwendeten Blasmodul Sterilisiereinrichtungen angeordnet sind. Hieraus resultiert ein sehr großer maschinenbaulicher Aufwand.

In der WO 2010/020530 A1 wird die Anordnung einer Sterilisiereinrichtung zwischen einer Heizung und dem Blasmodul beschrieben. Bei diesem Verfahren ist die Eintragsmenge von Sterilisationsmittel in den Bereich des Blasmoduls nur schwer vorhersehbar. Darüber hinaus ist die Austrittsmenge an Sterilisationsmittel in die Umgebung nicht kontrollierbar und eine entsprechende Kontamination nicht ausgeschlossen.

Nach einer erfolgten Sterilisierung und Beheizung der Vorformlinge werden diese einer Blasstation zugeführt und dort unter Verwendung von steriler Blasluft in die Behälter umgeformt. Die Zuführung der Blasluft und die Einspeisung der Blasluft erfolgt mittels einer Blasdüse, die z.B. dichtend an dem Vorformling anliegt. Bei der Blasformung der Behälter strömt die verwendete Blasluft z.B. aus der Reckstange heraus oder an der Reckstange vorbei. Darüber hinaus kommt die Reckstange im Bereich der Reckstangenkuppe sowohl mit dem Vorformling als auch mit dem geblasenen Behälter in Kontakt. Zur Gewährleistung einer ausreichenden Sterilität der geblasenen Behälter ist es somit erforderlich, auch für eine ausreichende Sterilität der Reckstange zu sorgen. Auch die Blasdüse sollte steril gehalten werden, um eine Verkeimung der Vorformlinge zu vermeiden. Ein zuverlässiges und zugleich technisch einfach durchführbares Verfahren hierfür ist bislang nicht bekannt geworden.

Verfahren und Vorrichtungen nach den Oberbegriffen der unabhängigen Ansprüchen werden in DE102007017938A1 und EP2483052B1 offenbart. Z

Aufgabe der vorliegenden Erfindung ist es, ein Verfahren der einleitend genannten Art derart zu verbessern, dass in einfacher Weise eine ausreichende Sterilität gewährleistet werden kann.

Diese Aufgabe wird mit den Merkmalen der unabhängigen Ansprüchen gelöst.

Weitere Aufgabe der vorliegenden Erfindung ist es, eine Vorrichtung der einleitend genannten Art derart zu konstruieren, dass eine effektive Sterilität mit geringem Aufwand gewährleistet ist.

Weitere vorteilhafte Ausgestaltungen des Verfahrens und der Vorrichtung sind in den Unteransprüchen angegeben.

Durch die Sterilisierung der Reckstange und/oder der Blasdüse im Bereich der Blasstation ist es möglich, arbeitsintensive Ein- und Ausbauvorgänge zu vermeiden, die für eine externe Sterilisierung der Reckstange oder der Blasdüse erforderlich wären. Die Verwendung von sterilisierender Strahlung in der Blasstation hat den weiteren Vorteil, dass eine berührungslose Bestrahlung der zu sterilisierenden Bereiche möglich ist, die insbesondere den Blasprozess nicht stört. Es ist lediglich erforderlich, Strahlungsquellen an geeigneten Stellen in der Blasstation anzuordnen, auf die zu sterilisierenden Bereiche auszurichten und an Versorgungsleitungen anzuschließen. Ein weiterer Vorteil der vorliegenden Erfindung ist, dass die Strahler die sterilisierende Strahlung permanent abgeben können, worunter auch die gepulste Strahlungsabgabe verstanden sein soll. Gerade bei der Sterilhaltung von Reckstange, Blasdüse oder Vorformling ist dies ein eminenter Vorteil, da z.B. während der gesamten Laufzeit der Blasmaschine die Strahler eingeschaltet sein können.

Für die Sterilhaltung ist es vorteilhaft, dass die Strahler auch während einer zeitweiligen Blasprozessunterbrechung in Betrieb bleiben. Solche Unterbrechungen, in denen die Blasmaschine nicht heruntergefahren wird, werden als Inline-Betrieb bezeichnet. Während dieses Inline-Betriebes können die Strahler weiterhin ihre keimtötende, sterilhaltende Wirkung entfalten. Für die Sterilisierung von Reckstange und Blasdüse während des Hochfahrens der Blasmaschine ist von Vorteil, dass auch während dieser Zeit die Strahler in Betrieb sind und sterilisierende Strahlung abstrahlen. Sterilhaltung meint die Verhinderung der Neuverkeimung eines bereits sterilisierten Bereiches, während Sterilisierung die Beseitigung vorhandener Keime meint.

Als Strahlungsquellen kommen verschiedene Strahler in Frage. Bevorzugte Strahlungsquellen sind aber UV-Strahler, die sich gegenüber alternativen Strahlungsquellen wie z.B. Elektronenstrahlern, Mikrowellenstrahlern oder Röntgenstrahlern dadurch auszeichnen, dass sie technisch einfacher in der Handhabung sind und weniger Aufwand bei der Abschirmung zu betreiben ist. Geeignete UV-Strahler sind im Stand der Technik bekannt, z.B. UV-LED's, Amalgam-Niederdrucklampen, Quecksilberdampflampen (Niederdruck, Mitteldruck, Hochdruck und Höchstdruck), Excimer-Laser, Diodenlaser.

Es werden bevorzugt UV-Strahler als Strahlungsquellen angeordnet, die insbesondere in einem für die Sterilisierung geeigneten Wellenlängenbereich Strahlung emittieren, z.B. im Bereich von 180-300 nm, sei es schmal- oder breitbandig, sei es gepulst oder im Daueremissionsbetrieb. Es wird als optimal angesehen, wenn die Strahlung intensitätsstark im Bereich um 220 nm und/oder 265 nm ist.

Mit Vorteil wird vorgeschlagen, dass die Reckstange aus einem UV-Strahlung leitenden Material ausgebildet ist, insbesondere aus einem Quarzglas, und UV-Strahlung in die Reckstange eingestrahlt, von der Reckstange bis in den Vorformling geleitet und auf die Vorformlingsinnenwand abgestrahlt wird. Dadurch wird nicht nur die Reckstange sterilisiert bzw. steril gehalten, sondern auch die Vorformlingsinnenwand des Vorformlings. Geeignete Gläser sind im Stand der Technik bekannt, z.B. aus der DE 10 2009 015 088 A1.

Dies wird noch dadurch in vorteilhafter Weise unterstützt, dass die Reckstange wenigstens einen inneren Kanal aufweist, und ionisierte Luft und/oder ein chemisches Sterilisationsmittel, insbesondere Wasserstoffperoxid, durch den inneren Kanal hindurch in den Vorformling hinein- und/oder aus dem Vorformling herausgeführt wird. Alternativ oder zusätzlich ist es bei Verwendung von UV-Strahlern vorteilhaft, dass die Vorformlinge durch den Innenkanal mit Stickstoff gespült werden, um Sauererstoff aus den Vorformlingen zu entfernen, da anderenfalls bei Bestrahlung mit Wellenlängen unterhalb von etwa 200 nm Ozon gebildet werden würde.

Bezüglich der Anordnung der Strahlungsquelle ergeben sich verschiedene Möglichkeiten. So kann eine Strahlungsquelle z.B. bezüglich der Blasstation höhenfest angeordnet und die Reckstange und/oder die Blasdüse bei ihrer Höhenpositionierbewegung an der Strahlungsquelle vorbeibewegt werden. Dies hat zum einen den Vorteil, dass keine Relativbewegung der Strahlungsquelle zur Blasstation erforderlich ist und dadurch bedingte konstruktive Komplikationen vermieden werden. Zum anderen wird dadurch ein größerer Bereich mit Strahlung abgedeckt, als bei einer ortsfesten Anordnung der Strahlungsquelle relativ zur Reckstange oder zur Blasdüse.

Mit Vorteil wird dabei die Strahlungsquelle positionsfest in der Blasstation angeordnet, z.B. an einem die Blasform tragenden Rahmen. Dies hat konstruktive Vorteile gegenüber einer Anordnung an beweglichen Teilen, was aber grundsätzlich auch möglich ist. Die Anordnung und Ausrichtung ist dabei so zu wählen, dass die Strahlungsquelle auf die Reckstange, auf die dem Vorformling zugewandte und ggf. auf den Vorformling zur Anlage kommende Seite der Blasdüse und/oder auf den Mündungsbereich des Vorformlings abstrahlt.

Für den Fall, dass mehrere Blasstationen auf einem rotierenden Blasrad angeordnet werden, ist es von Vorteil, dass jede Blasstation eine mitrotierende Sterilisationseinrichtung aufweist. Vorteile werden sowohl in konstruktiver Hinsicht als auch in der Einwirkung der sterilisierenden Strahlung gesehen (längere Einwirkdauer und geringere Entfernung der Strahlungsquelle). Eine weniger bevorzugte Alternative wäre z.B., dass feststehende Strahlungsquellen angeordnet werden, an denen die Blasstationen vorbeirotieren.

Eine bevorzugte Anordnungsmöglichkeit besteht dabei darin, dass eine Strahlungsquelle an der Blasdüse angeordnet wird, und Strahlung auf die Reckstange und/oder auf den Mündungsbereich des Vorformlings und/oder auf den mit dem Vorformling in Berührung kommenden Blasdüsenbereich abstrahlt. Die Strahlungsquelle sitzt dadurch sehr nahe zu dem Bereich, an dem die Strahlung ihre sterilisierende Wirkung entfalten soll. Ein weiterer Vorteil wird darin gesehen, dass das Ausrichten der Strahlungsquelle bei dieser Anordnung an der Blasdüse einfach ist.

Es wird weiterhin mit Vorteil vorgeschlagen, dass die Strahlungsquelle zentrosymmetrisch, insbesondere ringförmig, ausgebildet ist, den zu sterilisierenden Bereich ringförmig umgibt und die sterilisierende Strahlung ins Ringinnere abgestrahlt wird. Damit ist z.B. auch eine Anordnung gemeint, bei der die Strahlungsquelle aus mehreren Quellen besteht, z.B. aus drei oder mehr Strahlungsquellen, die auf einem Radius angeordnet sind, in dessen Mittelpunkt z.B. die Reckstange oder die Blasdüse sitzen. Mit Vorteil lassen sich dadurch Blasdüse, Reckstange und/oder Vorformling allseitig und vollumfänglich bestrahlen, ohne dass Umfangsbereiche ausgelassen sind. Mit Vorteil sind die Strahlungsquellen dazu ringförmig und äquidistant umfangsverteilt angeordnet. Eine ringförmig ausgebildete Strahlungsquelle erreicht diese Vorteile ebenfalls.

Es wird weiterhin mit Vorteil vorgeschlagen, dass um die Blasdüse herum, insbesondere an der Blasdüse entlang, und/oder von der Blasdüse ausgehend und in Richtung auf den Vorformling Sterilluft zur Ausbildung eines den Vorformlung umgebenden Sterilluftschleiers abgeblasen wird, insbesondere laminar strömend. Die Gefahr einer Neuverkeimung kann durch diesen Sterilluftschleier, der um den Vorformling herum ausgebildet wird, wirkungsvoll weiter verringert werden, da Keime nicht in den Vorformling eindringen oder sich an ihm anlagern können.

Die oben für erfindungsgemäße Verfahren geschilderten Vorteile gelten in analoger Weise für die erfindungsgemäßen Vorrichtungen.

in den Zeichnungen sind Ausführungsbeispiele der Erfindung schematisch dargestellt. Es zeigen:
- Fig. 1: eine perspektivische Darstellung einer Blasstation zur Herstellung von Behältern aus Vorformlingen,
- Fig. 2: einen Längsschnitt durch eine Blasform, in der ein Vorformling gereckt und expandiert wird,
- Fig. 3: eine Skizze zur Veranschaulichung eines grundsätzlichen Aufbaus einer Vorrichtung zur Blasformung von Behältern,
- Fig.4: eine modifizierte Heizstrecke mit vergrößerter Heizkapazität,
- Fig.5: eine schematische Darstellung eines Vorformlinges mit darin eingefahrener Reckstange nach einem ersten und einem zweiten Ausführungsbeispiel,
- Fig.6: eine schematische Darstellung eines in einer Blasform aufgenommenen Vorformlinges mit eingefahrener Reckstange und mit zur Blasluftbeaufschlagung angeordneter Blasdüse,
- Fig. 7: in schematischer Darstellung zwei beispielhaft gezeigte Blasstationen auf einem rotierenden Blasrad mit Sterilisierungseinrichtung nach einem ersten und einem zweiten Ausführungsbeispiel, und
- Fig.8: in einer schematischen Schnittdarstellung eine Anordnung der Sterilisierungseinrichtung innerhalb einer Blasstation.

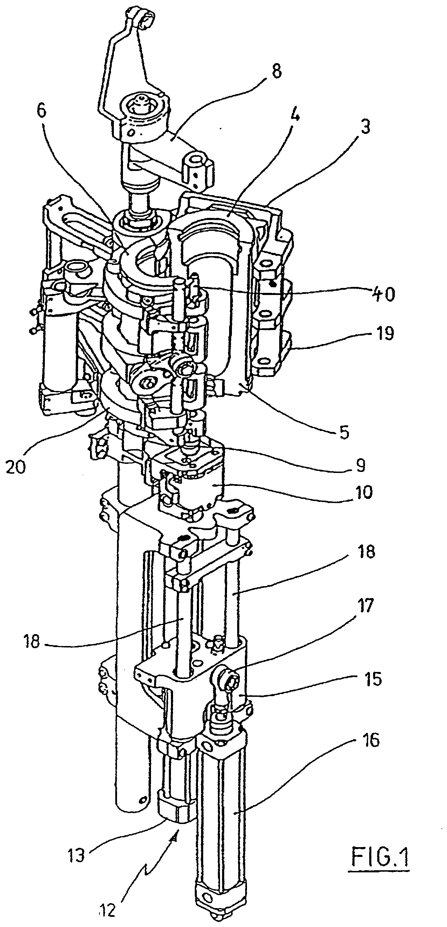

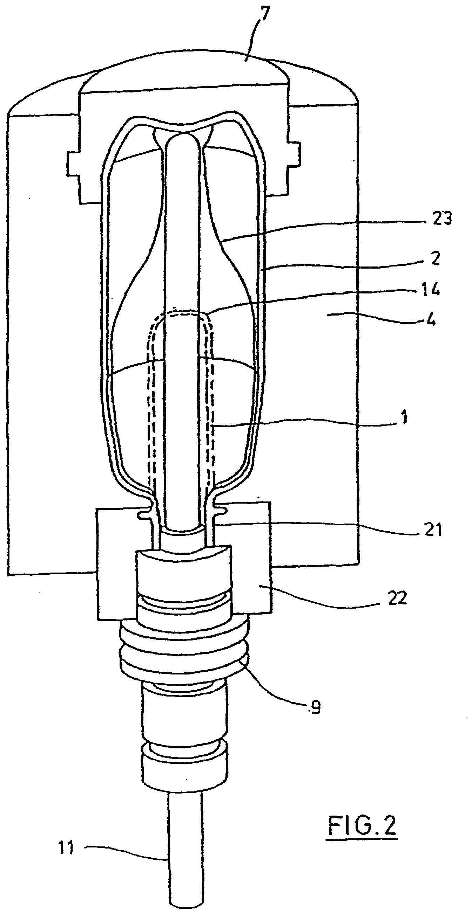

Der prinzipielle Aufbau einer Vorrichtung zur Umformung von Vorformlingen 1 in Behälter 2 ist in Fig. 1 und in Fig. 2 dargestellt.

Die Vorrichtung zur Formung des Behälters 2 besteht im wesentlichen aus einer Blasstation 3, die mit einer Blasform 4 versehen ist, in die ein Vorformling 1 einsetzbar ist. Der Vorformling 1 kann ein spritzgegossenes Teil aus Polyethylenterephthalat sein. Zur Ermöglichung eines Einsetzens des Vorformlings 1 in die Blasform 4 und zur Ermöglichung eines Herausnehmens des fertigen Behälters 2 besteht die Blasform 4 aus Formhälften 5, 6 und einem Bodenteil 7, das von einer Hubvorrichtung 8 positionierbar ist. Der Vorformling 1 kann im Bereich der Blasstation 3 von einem Transportdorn 9 gehalten sein, der gemeinsam mit dem Vorformling 1 eine Mehrzahl von Behandlungsstationen innerhalb der Vorrichtung durchläuft. Es ist aber auch möglich, den Vorformling 1 beispielsweise über Zangen oder andere Handhabungsmittel direkt in die Blasform 4 einzusetzen.

Zur Ermöglichung einer Druckluftzuleitung ist unterhalb des Transportdornes 9 eine Blasdüse 10 angeordnet, die dem Vorformling 1 Druckluft zuführt und gleichzeitig eine Abdichtung relativ zum Transportdorn 9 vornimmt. Bei einer abgewandelten Konstruktion ist es grundsätzlich aber auch denkbar, feste Druckluftzuleitungen zu verwenden.

Eine Reckung des Vorformlings 1 erfolgt mit Hilfe einer Reckstange 11, die von einem Zylinder 12 positioniert wird. Grundsätzlich ist es aber auch denkbar, eine mechanische Positionierung der Reckstange 11 über Kurvensegmente durchzuführen, die von Abgriffrollen beaufschlagt sind. Die Verwendung von Kurvensegmenten ist insbesondere dann zweckmäßig, wenn eine Mehrzahl von Blasstationen 3 auf einem rotierenden Blasrad angeordnet sind. Eine Verwendung von Zylindern 12 ist zweckmäßig, wenn ortsfest angeordnete Blasstationen 3 vorgesehen sind.

Bei der in Fig. 1 dargestellten Ausführungsform ist das Recksystem derart ausgebildet, dass eine Tandem-Anordnung von zwei Zylindern 12 bereitgestellt ist. Von einem Primärzylinder 13 wird die Reckstange 11 zunächst vor Beginn des eigentlichen Reckvorganges bis in den Bereich eines Bodens 14 des Vorformlings 1 gefahren. während des eigentlichen Reckvorganges wird der Primärzylinder 13 mit ausgefahrener Reckstange gemeinsam mit einem den Primärzylinder 13 tragenden Schlitten 15 von einem Sekundärzylinder 16 oder über eine Kurvensteuerung positioniert. Insbesondere ist daran gedacht, den Sekundärzylinder 16 derart kurvengesteuert einzusetzen, dass von einer Führungsrolle 17 , die während der Durchführung des Reckvorganges an einer Kurvenbahn entlang gleitet, eine aktuelle Reckposition vorgegeben wird. Die Führungsrolle 17 wird vom Sekundärzylinder 16 gegen die Führungsbahn gedrückt. Der Schlitten 15 gleitet entlang von zwei Führungselementen 18 . Nach einem Schließen der im Bereich von Trägern 19, 20 angeordneten Formhälften 5, 6 erfolgt eine Verriegelung der Träger 19, 20 relativ zueinander mit Hilfe einer Verriegelungseinrichtung 40.

Zur Anpassung an unterschiedliche Formen eines Mündungsabschnittes 21 des Vorformlings 1 ist gemäß Fig. 2 die Verwendung separater Gewindeeinsätze 22 im Bereich der Blasform 4 vorgesehen.

Fig. 2 zeigt zusätzlich zum geblasenen Behälter 2 auch gestrichelt eingezeichnet den Vorformling 1 und schematisch eine sich entwickelnde Behälterblase 23 .

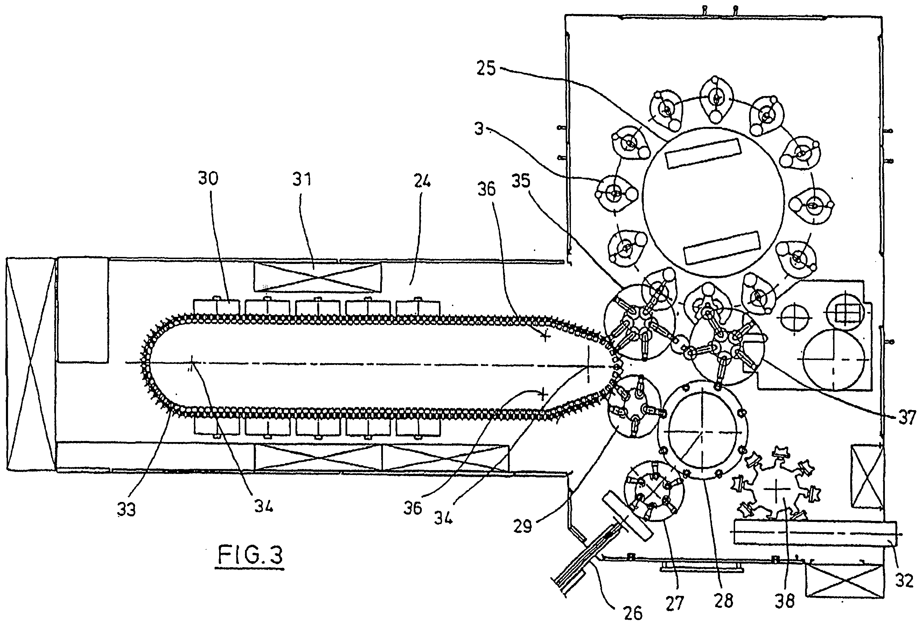

Fig. 3 zeigt den grundsätzlichen Aufbau einer Blasmaschine, die mit einer Heizstrecke 24 sowie einem rotierenden Blasrad 25 versehen ist. Ausgehend von einer Vorformlingseingabe 26 werden die Vorformlinge 1 von Übergaberädern 27, 28, 29 in den Bereich der Heizstrecke 24 transportiert. Entlang der Heizstrecke 24 sind Heizstrahler 30 sowie Gebläse 31 angeordnet, um die Vorformlinge 1 zu temperieren. Nach einer ausreichenden Temperierung der Vorformlinge 1 werden diese an das Blasrad 25 übergeben, in dessen Bereich die Blasstationen 3 angeordnet sind. Die fertig geblasenen Behälter 2 werden von weiteren Übergaberädern einer Ausgabestrecke 32 zugeführt.

Um einen Vorformling 1 derart in einen Behälter 2 umformen zu können, dass der Behälter 2 Materialeigenschaften aufweist, die eine lange Verwendungsfähigkeit von innerhalb des Behälters 2 abgefüllten Lebensmitteln, insbesondere von Getränken, gewährleisten, müssen spezielle Verfahrensschritte bei der Beheizung und Orientierung der Vorformlinge 1 eingehalten werden. Darüber hinaus können vorteilhafte Wirkungen durch Einhaltung spezieller Dimensionierungsvorschriften erzielt werden.

Als thermoplastisches Material können unterschiedliche Kunststoffe verwendet werden. Einsatzfähig sind beispielsweise PET, PEN oder PP.

Die Expansion des Vorformlings 1 während des Orientierungsvorganges erfolgt durch Druckluftzuführung. Die Druckluftzuführung ist in eine Vorblasphase, in der Gas, zum Beispiel Pressluft, mit einem niedrigen Druckniveau zugeführt wird und in eine sich anschließende Hauptblasphase unterteilt, in der Gas mit einem höheren Druckniveau zugeführt wird. Während der Vorblasphase wird typischerweise Druckluft mit einem Druck im Intervall von 10 bar bis 25 bar verwendet und während der Hauptblasphase wird Druckluft mit einem Druck im Intervall von 25 bar bis 40 bar zugeführt.

Aus Fig. 3 ist ebenfalls erkennbar, dass bei der dargestellten Ausführungsform die Heizstrecke 24 aus einer Vielzahl umlaufender Transportelemente 33 ausgebildet ist, die kettenartig aneinandergereiht und entlang von Umlenkrädern 34 geführt sind. Insbesondere ist daran gedacht, durch die kettenartige Anordnung eine im Wesentlichen rechteckförmige Grundkontur aufzuspannen. Bei der dargestellten Ausführungsform werden im Bereich der dem Übergaberad 29 und einem Eingaberad 35 zugewandten Ausdehnung der Heizstrecke 24 ein einzelnes relativ groß dimensioniertes Umlenkrad 34 und im Bereich von benachbarten Umlenkungen zwei vergleichsweise kleiner dimensionierte Umlenkräder 36 verwendet. Grundsätzlich sind aber auch beliebige andere Führungen denkbar.

Zur Ermöglichung einer möglichst dichten Anordnung des Übergaberades 29 und des Eingaberades 35 relativ zueinander erweist sich die dargestellte Anordnung als besonders zweckmäßig, da im Bereich der entsprechenden Ausdehnung der Heizstrecke 24 drei Umlenkräder 34, 36 positioniert sind, und zwar jeweils die kleineren Umlenkräder 36 im Bereich der Überleitung zu den linearen Verläufen der Heizstrecke 24 und das größere Umlenkrad 34 im unmittelbaren Übergabebereich zum Übergaberad 29 und zum Eingaberad 35 . Alternativ zur Verwendung von kettenartigen Transportelementen 33 ist es beispielsweise auch möglich, ein rotierendes Heizrad zu verwenden.

Nach einem fertigen Blasen der Behälter 2 werden diese von einem Entnahmerad 37 aus dem Bereich der Blasstationen 3 herausgeführt und über das Übergaberad 28 und ein Ausgaberad 38 zur Ausgabestrecke 32 transportiert.

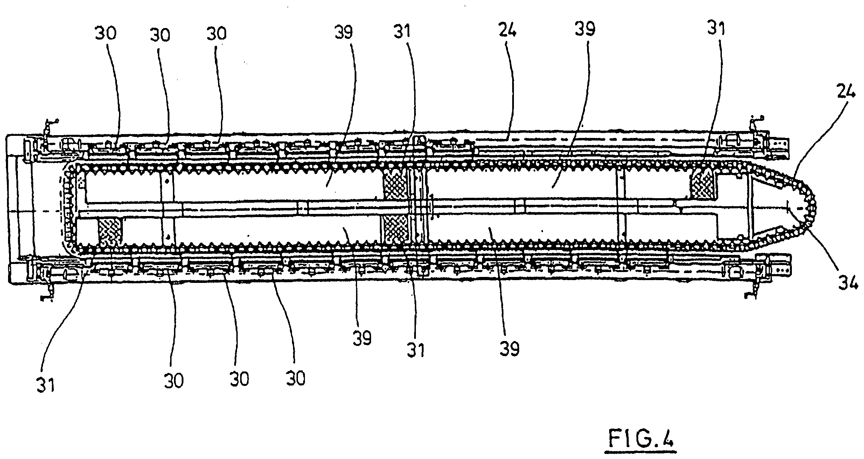

In der in Fig. 4 dargestellten modifizierten Heizstrecke 24 können durch die größere Anzahl von Heizstrahlern 30 eine größere Menge von Vorformlingen 1 je Zeiteinheit temperiert werden. Die Gebläse 31 leiten hier Kühlluft in den Bereich von Kühlluftkanälen 39 ein, die den zugeordneten Heizstrahlern 30 jeweils gegenüberliegen und über Ausströmöffnungen die Kühlluft abgeben. Durch die Anordnung der Ausströmrichtungen wird eine Strömungsrichtung für die Kühlluft im Wesentlichen quer zu einer Transportrichtung der Vorformlinge 1 realisiert. Die Kühlluftkanäle 39 können im Bereich von den Heizstrahlern 30 gegenüberliegenden Oberflächen Reflektoren für die Heizstrahlung bereitstellen, ebenfalls ist es möglich, über die abgegebene Kühlluft auch eine Kühlung der Heizstrahler 30 zu realisieren.

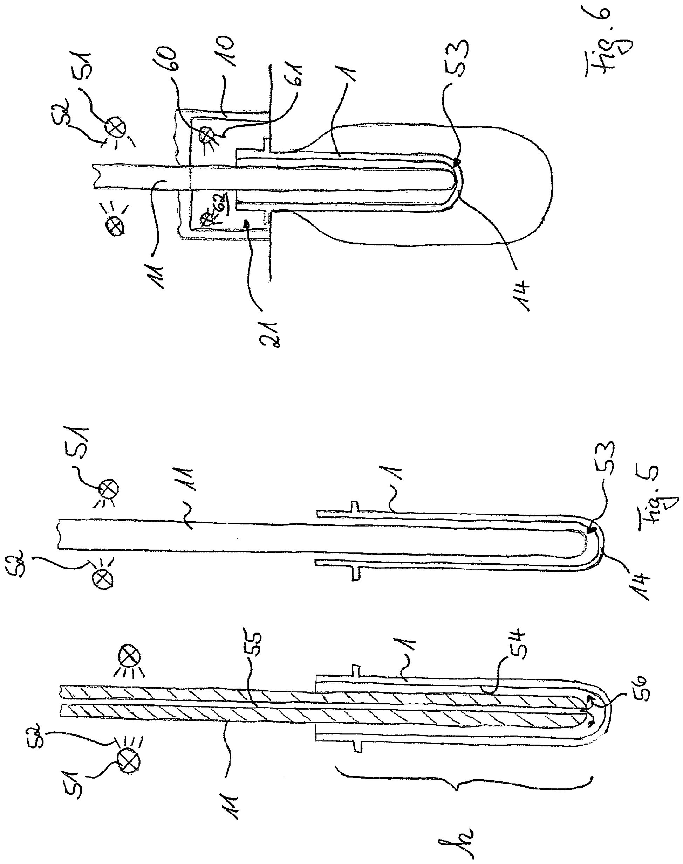

Fig. 5 zeigt schematisch und stark vereinfacht zwei Ausführungsbeispiele für die Ausbildung einer Sterilisationseinrichtung in einer Blasstation. Zur Vereinfachung der Darstellung wird lediglich der Vorformling 1 gezeigt. Nicht dargestellt ist insbesondere, dass dieser Vorformling 1 in einer Blasform aufgenommen ist. Weitere für die Erläuterung dieses Ausführungsbeispiels nicht relevante Teile der Blasstation sind ebenfalls zur Vereinfachung weggelassen.

Die rechte Hälfte der Fig. 5 zeigt eine Reckstange 11, die z.B. in bekannter Weise aus einem metallischen Material hergestellt ist. Zur Reckung des Vorformlings 1 wird die Reckstange 11 höhenpositioniert. Es ist dazu eine nicht dargestellte Hubvorrichtung vorgesehen, wie sie z.B. zu Fig. 1 erläutert wurde. In einem Höhenbereich der Reckstange 11 sind mehrere UV-Strahler 51 angeordnet, die UV-Strahlung 52 in Richtung auf die Reckstange 11 abstrahlen. Durch diese UV-Strahlung 52 wird die Reckstange 11 im bestrahlten Bereich entkeimt beziehungsweise keimfrei gehalten. Beim Reckvorgang bzw. während der Höhenpositionierung der Reckstange 11 wandert die Reckstange 11 durch die auf gegenüberliegenden Seiten angeordneten UV-Strahler 51 hindurch und es wird dadurch kontinuierlich ein neuer Bereich der Reckstange 11 mit UV-Strahlung 52 bestrahlt. Die Höhenpositionierung der UV-Strahler 51 ist dabei bevorzugt so gewählt, dass die Reckstange 11 im Laufe ihrer Höhenbewegung von der Reckstangenspitze 53 beginnend mit UV-Strahlung 52 überstrichen wird. Auf diese Weise wird der gesamte Bereich der Reckstange 11, der in den Vorformling 1 während des Reckprozesses eingefahren wird, zuverlässig mit UV-Strahlung 52 beaufschlagt. Der UV-Strahler 51 kann z.B. ringförmig ausgebildet sein. Es können aber auch mehrere, die Reckstange 11 ringförmig umgebende UV-Strahler 51 vorgesehen sein. Der dargestellte Zustand entspricht in etwa dem Zeitpunkt, zu dem die Reckstangenspitze 53 in Anlage zum Vorformlingsboden 14 kommt und eine Reckkraft auszuüben beginnt.

Die linke Hälfte der Fig. 5 zeigt eine gegenüber der rechten Hälfte abgewandelte Ausführungsform der Erfindung. In dieser linken Hälfte ist die Reckstange 11 aus einem Material hergestellt, das UV-Strahlung leitet. Gedacht ist insbesondere an die Ausbildung aus einem Quarzglas. Wie auch schon bezüglich der rechten Hälfte der Fig. 5 ausgeführt, sind auf einem Höhenbereich der Reckstange 11 UV-Strahler 51 angeordnet, die UV-Strahlung 52 in Richtung auf die Reckstange 11 abstrahlen. Aufgrund der Ausbildung der Reckstange z.B. aus einem Quarzglas kann die UV-Strahlung 52 in die Reckstange 11 eintreten. Die UV-Strahlung 52 wird innerhalb der Reckstange 11 geführt, insbesondere in Richtung auf den Vorformling 1, und tritt über die Reckstangenoberfläche und über einen Höhenbereich h der Reckstange 11 aus dieser aus und trifft insbesondere auf die innere Oberfläche 54 des Vorformlings 1.

Die Führung der UV-Strahlung 52 innerhalb der Reckstange 11 kann dadurch unterstützt werden, dass nicht dargestellte Einkoppelmittel vorgesehen sind, um die UV-Strahlung 52 gezielt in die Reckstange 11 einzukoppeln. Es könnte z.B. in einem Einkoppelbereich ein reflektierender Spiegel, ein Prisma oder dergleichen angeordnet werden. Die UV-Strahlung 52 könnte z.B. auch mittels UV-Lichtleitfasern von außen in die Reckstange 11 zugeführt werden. Es können auch die Abstrahlung von UV-Strahlung fördernde Mittel in der Reckstange 11 oder an der Reckstange vorgesehen sein. Es können z.B. über einen Längenbereich h der Reckstange 11 im Reckstangenmaterial Streukörper vorgesehen sein oder die Oberfläche der Reckstange 11 kann durch einen Facettenschliff zur gezielten Abstrahlung ausgebildet sein. Bevorzugt strahlt die Reckstange 11 über einen Längenbereich h UV-Strahlung 52 ab, der von der Reckstangenspitze 53 beginnend bis zu einer Höhe verläuft, die der Höhe entspricht, bis zu der die Reckstange 11 beim Einfahren in den Vorformling 1 bis zur Anlage an den Bodenbereich 14 des Vorformlings 1 eintaucht. Der Abstrahlbereich kann aber auch länger sein, um z.B. während des Reckprozesses möglichst über den ganzen Höhenbereich des sich entwickelnden Behälters 2 UV-Strahlung 52 abzugeben. Weiterhin in Abwandlung zur rechten Hälfte der Fig. 5 weist die Reckstange 11 in der linken Hälfte der Fig. 5 eine zentrale Bohrung 55 auf, durch die hindurch z.B. ionisierte Luft 56 in den Vorformling 1 eingeblasen werden kann, um Verunreinigungen aus dem Vorformling 1 herauszublasen. Durch diesen inneren Kanal 55 kann auch Wasserstoffperoxid 57 oder ein anderes geeignetes chemisches Sterilisierungsmittel z.B. in gasförmigem Aggregatzustand in den Vorformling 1 eingeführt werden, um eine zusätzliche Sterilisierung herbeizuführen. Es ist auch denkbar, diesen inneren Kanal 55 nicht für das Zuführen der genannten Medien zu verwenden, sondern für das Abführen, d.h. die ionisierte Luft 56 beziehungsweise das gasförmige Wasserstoffperoxid 57 könnte durch diesen inneren Kanal 55 aus dem Vorformling 1 abgeführt werden. In nicht dargestellter Weise kann die Reckstange 11 auch mehrere innere Kanäle 55 aufweisen, die dann z.B. sowohl für das Zuführen als auch für das Abführen der Medien verwendbar sind. Es ist auch möglich, durch einen der Kanäle 55 ionisierte Luft 56 und durch einen anderen der Kanäle 55 Wasserstoffperoxid 57 zuzuführen beziehungsweise abzuführen. Es ist auch denkbar, alle Kanäle 55 in gleicher Weise für die Zuführung und/oder für die Abführung der genannten Medien zu verwenden. Diese Varianten sind alle auch auf die Reckstange 11 der rechten Bildhälfte übertragbar.

Fig. 6 zeigt in einer stark schematisierten Darstellung ein weiteres Ausführungsbeispiel der Erfindung. Es ist eine Blasdüse 10 gezeigt, die der Zuführung der Blasluft dient, wie dies z.B. anhand der Fig. 1 bereits erläutert wurde. Erfindungsgemäß ist vorgesehen, dass innerhalb der Blasdüse 10 UV-Strahler 60 angeordnet sind. Diese UV-Strahler 60 sind dabei so angeordnet, dass die Abstrahlung des UV-Strahlung 61 in Richtung auf die Reckstange 11 erfolgt. Gleichzeitig wird auch der Neckbereich 21 des Vorformlings 1 bestrahlt, und auch der Innenraum 62 der Blasdüse 10 wird mit UV-Strahlung 61 beaufschlagt. Auch hier können die UV-Strahler 60 ringförmig angeordnet oder ringförmig ausgebildet sein. Aufgrund der Relativbewegung zwischen Blasdüse 10 und Reckstange 11 wird ein bestimmter Längenbereich der Reckstange 11 mit UV-Strahlung 61 beleuchtet, der von der Reckstangenspitze 53 beginnend bis hin zu einer Endstellung der Reckstange 11 verläuft. Zusätzlich sind außerhalb und oberhalb der Blasdüse 10 weitere UV-Strahler 51 angeordnet, die in Richtung auf die Reckstange 11 UV-Strahlung 52 abstrahlen. Hierzu kann auf Fig. 5 verwiesen werden.

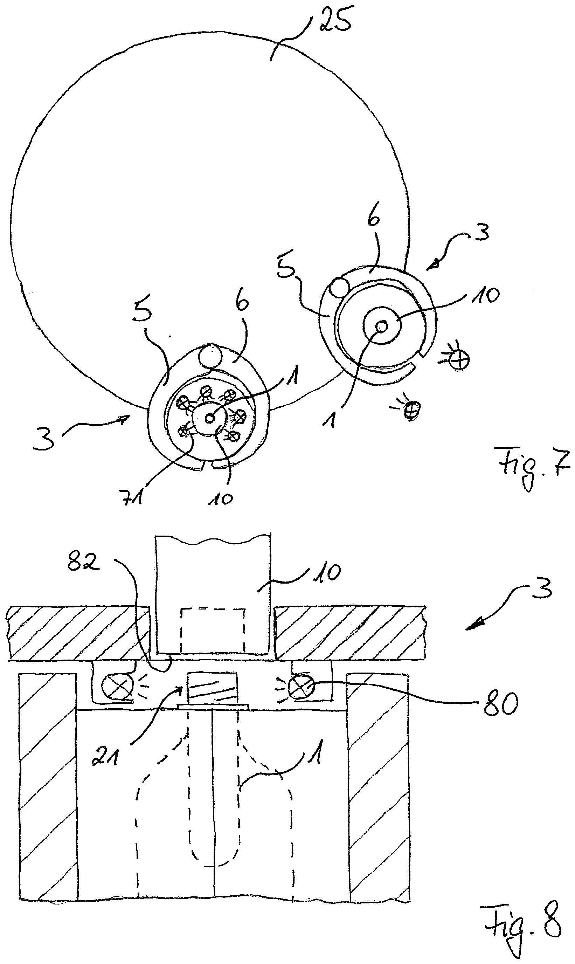

Fig. 7 zeigt in stark schematisierter Darstellung ein Blasrad 25 mit zwei exemplarisch dargestellten Blasstationen 3 mit jeweils zwei Blasformhälften 5, 6. Die in der 6-Uhr-Position des Blasrades 25 gezeigte Blasstation 3 weist die Blasdüse 10 ringförmig umgebende UV-Strahler 70 auf. Diese Strahler 70 sind so angeordnet und ausgerichtet, dass die Blasdüse 10 mit UV-Strahlung 71 beaufschlagt wird. Bevorzugt sind die UV-Strahler 70 so ausgerichtet, dass der untere, dem Vorformling 1 zugewandte Bereich der Blasdüse 10 angestrahlt wird, insbesondere der mit dem Vorformling 1 in Kontakt gelangende Bereich. Sobald die Blasdüse 10 mit ihrer Absenkbewegung beginnt, um auf den Vorformling 1 dichtend abgesenkt zu werden, wird auch der benachbarte Bereich der Blasdüse 10 mit UV-Strahlung 71 bestrahlt.

Die in der 4-Uhr-Position des Blasrades 25 gezeigte Blasstation 3 weist UV-Strahler 73 auf, die stationär angeordnet und so höhenpositioniert und ausgerichtet sind, dass die sich vorbeibewegende Blasdüse 10 mit UV-Strahlung 74 bestrahlt wird. Auch hier erfolgt bevorzugt eine Bestrahlung der dem Vorformling 1 zugewandten Seite der Blasdüse 10.

Fig. 8 zeigt in einer schematischen Schnittdarstellung die Anordnung von UV-Strahlern 80 an einem feststehenden Teil der Blasstation 3. Es ist erkennbar, dass die UV-Strahlung 81 nicht nur die Unterseite 82 der Blasdüse 10 beaufschlagt, sondern auch den Neckbereich 21 des Vorformlings 1. Sobald die nicht dargestellte Reckstange durch die Blasdüse 10 hindurch in Richtung auf den Vorformling 1 positioniert wird, wird auch die Reckstange mit UV-Licht bestrahlt. Auf die geschilderte Weise werden alle Bereiche mit sterilisierender Strahlung überdeckt, die für eine zuverlässige Sterilisierung beziehungsweise Sterilhaltung relevant sind.

## Patentansprüche

1. Verfahren zur Herstellung von blasgeformten, mindestens bereichsweise sterilen Behältern (2) in einer Blasformungsmaschine, bei dem ein Vorformling (1) aus einem thermoplastischen Material zunächst erwärmt und dann in einer Blasstation (3) von einer Reckstange (11) gereckt und über eine Blasdüse (10) mit einem unter Druck stehenden Fluid beaufschlagt wird, sowie bei dem eine Sterilisationseinrichtung in der Blasstation (3) angeordnet wird, wobei die Sterilisationseinrichtung wenigstens eine Strahlungsquelle aufweist, die eine sterilisierende Strahlung auf die Reckstange (11) und/oder auf die Blasdüse (10) abstrahlt, wobei die Strahlungsquelle insbesondere eine UV-Strahlungsquelle ist und UV-Strahlung abstrahlt, **dadurch gekennzeichnet, dass** die Strahlungsquelle an der Blasdüse (10) angeordnet wird, und Strahlung auf die Reckstange (11) und/oder auf den Mündungsbereich (21) des Vorformlings (1) und/oder auf den mit dem Vorformling (1) in Berührung kommenden Blasdüsenbereich abstrahlt.

2. Verfahren nach Anspruch 1, **dadurch gekennzeichnet, dass** die sterilisierende Strahlung während des Blasformungsprozesses und/oder während des Inline-Betriebes und/oder während des Hochfahrens der Blasformungsmaschine abgestrahlt wird.

3. Verfahren nach Anspruch 1, **dadurch gekennzeichnet, dass** die Reckstange (11) aus einem UV-Strahlung leitendem Material ausgebildet ist, insbesondere aus einem Quarzglas, und UV-Strahlung in die Reckstange (11) eingestrahlt, von der Reckstange (11) bis in den Vorformling (1) geleitet und auf die Vorformlingsinnenwand abgestrahlt wird.

4. Verfahren nach einem der vorhergehenden Ansprüche, **dadurch gekennzeichnet, dass** die Reckstange (11) wenigstens einen inneren Kanal aufweist, und ionisierte Luft und/oder ein chemisches Sterilisationsmittel durch den inneren Kanal hindurch in den Vorformling (1) hinein- und/oder aus dem Vorformling (1) herausgeführt wird.

5. Verfahren nach einem der vorhergehenden Ansprüche, **dadurch gekennzeichnet, dass** eine Strahlungsquelle bezüglich der Blasstation (3) höhenfest angeordnet wird und die Reckstange (11) und/oder die Blasdüse (10) bei ihrer Höhenpositionierbewegung an der Strahlungsquelle vorbeibewegt werden, wobei insbesondere eine Strahlungsquelle positionsfest zur Blasstation (3) angeordnet wird, und Strahlung auf die dem Vorformling (1) zugewandte Seite der Blasdüse (10) und/oder auf den Mündungsbereich (21) des Vorformlings (1) abstrahlt.

6. Verfahren nach Anspruch 5, **dadurch gekennzeichnet, dass** mehrere Blasstationen (3) auf einem rotierenden Blasrad (25) angeordnet werden, und jede Blasstation (3) eine mitrotierende Sterilisationseinrichtung aufweist.

7. Verfahren nach einem der vorhergehenden Ansprüche, **dadurch gekennzeichnet, dass** die Strahlungsquelle zentrosymmetrisch, nämlich ringförmig, ausgebildet ist, den zu sterilisierenden Bereich ringförmig umgibt und die sterilisierende Strahlung ins Ringinnere abgestrahlt wird.

8. Verfahren nach einem der vorhergehenden Ansprüche, **dadurch gekennzeichnet, dass** um die Blasdüse (10) herum, insbesondere an der Blasdüse (10) entlang und/oder von der Blasdüse (10) ausgehend und in Richtung auf den Vorformling (1) Sterilluft zur Ausbildung eines Sterilluftschleiers abgeblasen wird, insbesondere laminar strömend.

9. Vorrichtung zur Herstellung von blasgeformten, mindestens bereichsweise sterilen Behältern (2), die eine Heizstrecke (24) zur Temperierung von Vorformlingen (1) aus einem thermoplastischen Material und wenigstens eine Blasstation (3) zur Blasverformung der Vorformlinge (1) in Behälter (2) aufweist, wobei die Blasstation (3) eine Reckstange (11) zur Reckung des Vorformlings (1) und eine Blasdüse (10) zur Beaufschlagung des Vorformlings (1) mit einem unter Druck stehenden Fluid aufweist, sowie bei der eine Sterilisationseinrichtung in der Blasstation angeordnet ist, wobei die Sterilisationseinrichtung wenigstens eine Strahlungsquelle aufweist, die eine sterilisierende Strahlung auf die Reckstange (11) und/oder auf die Blasdüse (10) abstrahlt, wobei die Sterilisiereinrichtung insbesondere ansteuerbar ist, sterilisierende Strahlung während des Blasformungsprozesses und/oder während des Inline-Betriebes und/oder während des Hochfahrens der Vorrichtung abzustrahlen, und wobei die Strahlungsquelle insbesondere als eine UV-Strahlung abstrahlende UV-Strahlungsquelle ausgebildet ist, wobei die Reckstange (11) insbesondere aus einem UV-Strahlung leitenden Material ausgebildet ist, insbesondere aus einem Quarzglas, **dadurch gekennzeichnet, dass** die Strahlungsquelle an der Blasdüse (10) so angeordnet ist, dass sie Strahlung auf die Reckstange (11) und/oder auf den Mündungsbereich (21) des Vorformlings (1) und/oder auf den mit dem Vorformling (1) in Berührung kommenden Blasdüsenbereich abstrahlt.

10. Vorrichtung nach Anspruch 9, **dadurch gekennzeichnet, dass** die Reckstange (11) wenigstens einen inneren Kanal aufweist, der mit einer Quelle oder Senke für ionisierte Luft und/oder mit einer Quelle oder Senke für ein chemisches Sterilisationsmittel in ventilbeherrschter Verbindung steht, um ionisierte Luft und/oder chemisches Sterilisationsmittel durch den inneren Kanal hindurch in den Vorformling (1) hinein- und/oder aus dem Vorformling (1) herauszuführen.

11. Vorrichtung nach einem der vorhergehenden Vorrichtungsansprüche, **dadurch gekennzeichnet, dass** eine Strahlungsquelle bezüglich der Blasstation (3) höhenfest so angeordnet ist, dass die Reckstange (11) und/oder die Blasdüse (10) bei ihrer Höhenpositionierbewegung an ihr vorbeibewegt werden, wobei eine Strahlungsquelle insbesondere positionsfest zur Blasstation (3) so angeordnet ist, dass sie Strahlung auf die dem Vorformling (1) zugewandte Seite der Blasdüse (10) und/oder auf den Mündungsbereich (21) des Vorformlings (1) abstrahlt, wobei insbesondere mehrere Blasstationen (3) auf einem rotierenden Blasrad (25) angeordnet sind und jede Blasstation (3) eine mitrotierende Sterilisationseinrichtung aufweist.

12. Vorrichtung nach einem der vorhergehenden Vorrichtungsansprüche, **dadurch gekennzeichnet, dass** die Strahlungsquelle zentrosymmetrisch, nämlich ringförmig, ausgebildet ist, den zu sterilisierenden Bereich ringförmig umgibt und die sterilisierende Strahlung ins Ringinnere abstrahlt.

13. Vorrichtung nach einem der vorhergehenden Vorrichtungsansprüche, **dadurch gekennzeichnet, dass** die Vorrichtung im Bereich der Blasstation mit Sterilluft versorgte Sterilluftauslässe aufweist, die so angeordnet und ausgebildet sind, dass Sterilluft um die Blasdüse (10) herum, insbesondere an der Blasdüse (10) entlang, und/oder von der Blasdüse (10) ausgehend und in Richtung auf den Vorformling (1) zur Ausbildung eines Sterilluftschleiers abgeblasen wird, insbesondere laminar strömend.

## Claims

1. A method for producing blow-moulded containers (2), which are sterile at least in some areas, in a blow-moulding machine, in which method a preform (1) made of a thermoplastic material is initially heated and is then stretched by a stretching rod (11) in a blowing station (3) and a pressurised fluid is applied to it via a blowing nozzle (10), and in which method a sterilisation device is arranged in the blowing station (3), wherein the sterilisation device has at least one radiation source, which emits a sterilising radiation onto the stretching rod (11) and/or onto the blowing nozzle (10), wherein the radiation source is in particular a UV radiation source and emits UV radiation, **characterised in that**
the radiation source is arranged on the blowing nozzle (10) and emits radiation onto the stretching rod (11) and/or onto the opening area (21) of the preform (1) and/or onto the blowing nozzle area, which comes into contact with the preform (1).

2. The method according to claim 1, **characterised in that** the sterilising radiation is emitted during the blow-moulding process and/or during the inline operation and/or during the start-up of the blow-moulding machine.

3. The method according to claim 1, **characterised in that** the stretching rod (11) is made of a UV radiation conducting material, in particular of quartz glass, and UV radiation is irradiated into the stretching rod (11), is guided from the stretching rod (11) into the preform (1), and is emitted onto the internal preform wall.

4. The method according to any one of the preceding claims, **characterised in that** the stretching rod (11) has at least one internal duct, and ionised air and/or a chemical sterilisation agent is guided through the internal duct into the preform (1) and/or out of the preform (1).

5. The method according to any one of the preceding claims, **characterised in that** a radiation source is arranged so as to be fixed in height in relation to the blowing station (3), and the stretching rod (11) and/or the blowing nozzle (10) are moved past the radiation source in response to their height positioning movement, wherein a radiation source is arranged so as to be in particular positionally fixed in relation to the blowing station (3) and emits radiation onto the side of the blowing nozzle (10) facing the preform (1) and/or onto the opening area (21) of the preform (1).

6. The method according to claim 5, **characterised in that** several blowing stations (3) are arranged on a rotating blowing wheel (25), and each blowing station (3) has a co-rotating sterilisation device.

7. The method according to any one of the preceding claims, **characterised in that** the radiation source is formed so as to be centrically symmetrical, namely in a ring-shaped manner, surrounds the area to be sterilised in a ring-shaped manner, and the sterilising radiation is emitted into the ring interior.

8. The method according to any one of the preceding claims, **characterised in that** sterile air for forming a sterile air curtain is blown off, in particular so as to flow in a laminar manner, around the blowing nozzle (10), in particular along the blowing nozzle (10), and/or starting at the blowing nozzle (10) and in the direction of the preform (1).

9. A device for producing blow-moulded containers (2), which are sterile at least in some areas, which device has a heating section (24) for controlling the temperature of preforms (1) made of a thermoplastic material, and at least one blowing station (3) for blow-moulding the preforms (1) into containers (2), wherein the blowing station (3) has a stretching rod (11) for stretching the preform (10) and a blowing nozzle (10) for applying a pressurised fluid to the preform (1), and in which a sterilisation device is arranged in the blowing station, wherein the sterilisation device has at least one radiation source, which emits a sterilising radiation onto the stretching rod (11) and/or onto the blowing nozzle (10), wherein the sterilisation device can in particular be controlled to emit sterilising radiation during the blow-moulding process and/or during the inline operation and/or during the start-up of the device, and wherein the radiation source is formed in particular as a UV radiation-emitting UV radiation source, wherein the stretching rod (11) is made in particular of a UV radiation conducting material, in particular of a quartz glass, **characterised in that** the radiation source is arranged on the blowing nozzle (10) in such a way that it emits radiation onto the stretching rod (11) and/or onto the opening area (21) of the preform (1) and/or onto the blowing nozzle area, which comes into contact with the preform (1).

10. The device according to claim 9, **characterised in that** the stretching rod (11) has at least one internal duct, which is connected in a valve-controlled manner to a source or sink for ionised air and/or to a source or sink for a chemical sterilisation agent, so as to guide ionised air and/or chemical sterilisation agent through the internal duct into the preform (1) and/or out of the preform (1).

11. The device according to any one of the preceding device claims, **characterised in that** a radiation source is arranged so as to be fixed in height in relation to the blowing station (3), that the stretching rod (11) and/or the blowing nozzle (10) are moved past said radiation source in response to their height positioning movement, wherein a radiation source is arranged so as to be in particular positionally fixed in relation to the blowing station (3) in such a way that it emits radiation onto the side of the blowing nozzle (10) facing the preform (1) and/or onto the opening area (21) of the preform (1), wherein in particular several blowing stations (3) are arranged on a rotating blowing wheel (25), and each blowing station (3) has a co-rotating sterilisation device.

12. The device according to any one of the preceding device claims, **characterised in that** the radiation source is formed so as to be centrically symmetrical, namely in a ring-shaped manner, surrounds the area to be sterilised in a ring-shaped manner, and the sterilising radiation is emitted into the ring interior.

13. The device according to any one of the preceding device claims, **characterised in that,** in the area of the blowing station, the device has sterile air outlets, which are supplied with sterile air and which are arranged and formed in such a way that sterile air is blown off, in particular so as to flow in a laminar manner, around the blowing nozzle (10), in particular along the blowing nozzle (10), and/or starting at the blowing nozzle (10) and in the direction of the preform (1), for forming a sterile air curtain.

## Revendications

1. Procédé de fabrication de récipients (2) au moins partiellement stériles moulés par soufflage dans une machine de moulage par soufflage, dans le cadre duquel une préforme (1) en un matériau thermoplastique est tout d'abord chauffée puis, dans une station de soufflage (3), étirée par une barre d'étirage (11) et, par l'intermédiaire d'une buse de soufflage (10), soumise à l'action d'un fluide de soufflage sous pression, et dans le cadre duquel un dispositif de stérilisation est agencé dans la station de soufflage (3), le dispositif de stérilisation présentant au moins une source de rayonnement qui émet un rayonnement stérilisant vers la barre d'étirage (11) et/ou la buse de soufflage (10), la source de rayonnement étant notamment une source de rayons UV qui émet des rayons UV, **caractérisé en ce que** La source de rayonnement est agencée au niveau de la buse de soufflage (10) et émet un rayonnement vers la barre d'étirage (11) et/ou la section d'embouchure (21) de la préforme (1) et/ou la partie de la buse de soufflage qui entre en contact avec la préforme (1).

2. Procédé selon la revendication 1, **caractérisé en ce que** le rayonnement stérilisant est émis pendant le processus de soufflage et/ou pendant le fonctionnement en ligne et/ou pendant la mise en route de la machine de moulage par soufflage.

3. Procédé selon la revendication 1, **caractérisé en ce que** la barre d'étirage (11) est réalisée en un matériau conducteur de rayons UV et notamment en verre de quartz, et qu'un rayonnement UV est émis dans la barre d'étirage (11) et est conduit de la barre d'étirage (11) jusque dans la préforme (1) où il rayonne sur la paroi intérieure de la préforme.

4. Procédé selon l'une des revendications précédentes, **caractérisé en ce que** la barre d'étirage (11) présente au moins un canal interne par lequel de l'air ionisé et/ou un agent de stérilisation chimique est introduit dans la préforme (1) ou en est évacué.

5. Procédé selon l'une des revendications précédentes, **caractérisé en ce qu'**une source de rayonnement est agencée à une hauteur fixe par rapport à la station de soufflage (3) et que la barre d'étirage (11) et/ou la buse de soufflage (10) est, lors de son positionnement en hauteur, déplacée devant la source de rayonnement, une source de rayonnement étant notamment agencée à une hauteur fixe par rapport à la station de soufflage (3) et émettant un rayonnement vers le côté de la buse de soufflage (10) faisant face à la préforme (1) et/ou vers la section d'embouchure (21) de la préforme (1).

6. Procédé selon la revendication 5, **caractérisé en ce que** plusieurs stations de soufflage (3) sont agencées sur une roue de soufflage (25) en rotation, chaque station de soufflage (3) présentant un dispositif de stérilisation tournant avec elle.

7. Procédé selon l'une des revendications précédentes, **caractérisé en ce que** la source de rayonnement est de conformation centrosymétrique, à savoir annulaire, et entoure en anneau la zone à stériliser, le rayonnement stérilisant étant émis vers l'intérieur de l'anneau.

8. Procédé selon l'une des revendications précédentes, **caractérisé en ce que** de l'air stérile est soufflé autour de la buse de soufflage (10) et notamment le long de la buse de soufflage (10) et/ou partant de la buse de soufflage (10) en direction de la préforme (1) pour former un rideau d'air stérile, notamment sous forme d'un flux laminaire.

9. Dispositif de fabrication de récipients (2) au moins partiellement stériles moulés par soufflage présentant une section de chauffage(24) pour équilibrer la température de préformes (1) en un matériau thermoplastique et au moins une station de soufflage (3) pour la transformation par moulage par soufflage des préformes en récipients (2), la station de soufflage (3) présentant une barre d'étirage (11) pour l'étirage de la préforme (1) et une buse de soufflage (10) pour soumettre la préforme (1) à l'action d'un fluide sous pression, et lequel présente un dispositif de stérilisation agencé dans la station de soufflage, le dispositif de stérilisation présentant au moins une source de rayonnement qui émet un rayonnement stérilisant vers la barre d'étirage (11) et/ou la buse de soufflage (10), le dispositif de stérilisation pouvant notamment être commandé de façon à émettre un rayonnement stérilisant pendant le processus de soufflage et/ou pendant le fonctionnement en ligne et/ou pendant la mise en route du dispositif, et la source de rayonnement étant notamment conçue comme source de rayons UV qui émet des rayons UV, la barre d'étirage (11) étant notamment réalisée en un matériau conducteur de rayons UV et notamment en verre de quartz, **caractérisé en ce que** la source de rayonnement est agencée au niveau de la buse de soufflage (10) de façon à émettre un rayonnement vers la barre d'étirage (11) et/ou la section d'embouchure (21) de la préforme (1) et/ou la partie de la buse de soufflage qui entre en contact avec la préforme (1).

10. Dispositif selon la revendication 9, **caractérisé en ce que** la barre d'étirage (11) présente au moins un canal interne relié par une connexion commandée par vanne à une source ou une évacuation d'air ionisé et/ou à une source ou une évacuation d'agent de stérilisation chimique pour introduire par le canal interne de l'air ionisé et/ou un agent de stérilisation chimique dans la préforme (1) ou l'en évacuer.

11. Dispositif selon l'une des revendications précédentes concernant le dispositif, **caractérisé en ce qu'**une source de rayonnement est agencée à une hauteur fixe par rapport à la station de soufflage (3) de façon à ce que la barre d'étirage (11) et/ou la buse de soufflage (10) soient, lors de leur positionnement en hauteur, déplacées devant la source de rayonnement, une source de rayonnement étant notamment agencée à une hauteur fixe par rapport à la station de soufflage (3) de façon à émettre un rayonnement vers le côté de la buse de soufflage (10) faisant face à la préforme (1) et/ou vers la section d'embouchure (21) de la préforme (1), plusieurs stations de soufflage (3) étant notamment agencées sur une roue de soufflage (25) en rotation, chaque station de soufflage (3) présentant un dispositif de stérilisation tournant avec elle.

12. Dispositif selon l'une des revendications précédentes concernant le dispositif, **caractérisé en ce que** la source de rayonnement est de conformation centrosymétrique, à savoir annulaire, et entoure en anneau la zone à stériliser et émet le rayonnement stérilisant vers l'intérieur de l'anneau.

13. Dispositif selon l'une des revendications précédentes concernant le dispositif, **caractérisé en ce que** le dispositif présente dans la zone de la station de soufflage des sorties d'air stérile alimentées en air stérile agencées et conçues de façon à souffler l'air stérile autour de la buse de soufflage (10) et notamment le long de la buse de soufflage (10) et/ou partant de la buse de soufflage (10) en direction de la préforme (1) pour former un rideau d'air stérile, notamment sous forme d'un flux laminaire.
